# EUROPÄISCHE PATENTSCHRIFT

(11) **EP 3 069 578 B1**
(45) Veröffentlichungstag und Bekanntmachung des Hinweises auf die Patenterteilung: **24.10.2018**
(21) Anmeldenummer: 14815218.4
(22) Anmeldetag: 06.11.2014
(51) Int. Cl.: H05H 1/24, A61N 1/44

(54) **GERÄT ZUR BEHANDLUNG EINER KÖRPEROBERFLÄCHE EINES LEBENDEN KÖRPERS**
DEVICE FOR TREATING A BODY SURFACE OF A LIVING BODY
APPAREIL DE TRAITEMENT D'UNE SURFACE CORPORELLE D'UN ÊTRE VIVANT

(30) Priorität: 15.11.2013 DE 102013019057
(43) Veröffentlichungstag der Anmeldung: 21.09.2016
(73) Patentinhaber: Cinogy GmbH, 37115 Duderstadt (DE)
(72) Erfinder: WANDKE, Dirk, 37308 Heilbad Heiligenstadt (DE); TRUTWIG, Leonhard, 37115 Duderstadt (DE); STORCK, Karl-Otto, 37115 Duderstadt (DE); HAHNL, Mirko, 37339 Berlingerode (DE); PALLASKE, Frank, 09355 Gersdorf (DE); HÄRTLEIN, Steffen, 09123 Chemnitz (DE); DÄSCHLEIN, Georg, 16348 Wandlitz OT Klosterfelde (DE)
(74) Vertreter: Lins, Martina
(86) Internationale Anmeldenummer: PCT/DE2014/000572
(87) Internationale Veröffentlichungsnummer: WO 2015/070832

(56) Entgegenhaltungen:
- EP-A1- 2 170 022
- WO-A1-2011/076193
- CA-A1- 2 846 684
- DE-A1-102011 105 713
- US-A1- 2012 107 896

## Beschreibung

Die Erfindung betrifft ein Gerät zur Behandlung einer Körperoberfläche eines lebenden Körpers mittels eines dielektrisch behinderten Plasmas, mit einem flächigen, flexiblen Dielektrikum, das eine an eine Hochspannungsquelle angeschlossene Elektrode von der Körperoberfläche abschirmt und zur Anlage an der als Gegenelektrode fungierenden Körperoberfläche geeignet und mit einer strukturierten Oberfläche ausgebildet ist, die einen Gasraum für die Plasmaentladung zwischen Dielektrikum und Körperober-fläche ermöglicht.

Ein derartiges Gerät ist durch DE 10 2009 060 627 A1 bekannt. Das flächige, flexible Dielektrikum ist dabei in seinen Flächendimensionen größer ausgebildet als die mit der Hochspannungsquelle verbundene Elektrode, sodass das Dielektrikum die Elektrode sicher zur Körperoberfläche hin abschirmt, sodass ein zerstörender Funkenüberschlag auf die Hautoberfläche sicher vermieden wird. Dabei kann die Elektrode auch in das Dielektrikum allseitig eingebettet sein und lediglich mit einer Anschlussleitung aus dem Dielektrikum herausführen. In der Ausgestaltung des bekannten Geräts ist es möglich, das Dielektrikum flächig direkt auf die Körperoberfläche aufzulegen. Um dabei die Ausbildung der Plasmaentladung zu ermöglichen, ist die zur Körperoberfläche zeigende Oberfläche des Dielektrikums strukturiert, sodass sich zwischen Dielektrikum und Körperoberfläche Gasräume ausbilden, in denen die Plasmaentladung stattfinden kann.

Die Plasmabehandlung der Körperoberfläche kann bei einer gesunden Körperoberfläche aus kosmetischen Gründen erfolgen, beispielsweise um die Körperoberfläche zu desinfizieren und für eine anschließende Behandlung mit Wirkstoffen vorzubereiten, wodurch die Aufnahme der Wirkstoffe durch die Haut verbessert wird. Eine Plasmabehandlung der Körperoberfläche hat sich darüber hinaus insbesondere für die Förderung der Wundheilung, insbesondere bei chronischen Wunden, bewährt. Die Plasmabehandlung hat eine starke antimikrobielle Wirkung, sodass mittels der Plasmabehandlung die Wundbehandlung störende Effekte durch eine bakterielle Besiedlung unterbunden werden können. Zu den die Wundheilung störenden Keimen zählen insbesondere Staphylococcus aureus, Pseudomonas aeruginosa, E.coli und andere multiresistente Erreger. Weiterhin kann die Mikrozirkulation im Gewebeverbund durch das Einwirken des Plasmas positiv beeinflusst werden.

Für die Wundbehandlung, insbesondere chronischer Wunden, werden in herkömmlicher Technik Wundverbände benutzt, die nach den Prinzipien der feuchten Wundbehandlung sterile Wundauflagen aufweisen, die zur Keimreduktion mit verschiedenen antiseptischen Wirkstoffen versehen werden. Außerdem werden die Wunden antiseptisch gespült. Bei tiefen Belägen werden die Wunden zusätzlich mittels chirurgischer Verfahren gereinigt. Diese Verfahren führen häufig nur zu einer unzureichenden Keimreduktion, sodass sowohl weiter Infektionsrisiken als auch Keimverbreitungsrisiken bestehen. Bei starken Besiedlungen, insbesondere in Form eines Biofilms, sowie beim Auftreten von hochvirulenten Erregern, beispielsweise Gruppe-A-Streptokokken, wird die Wundheilung verzögert oder durch Infektionen überhaupt verhindert. Effektive Verfahren, die auf dem Einsatz chirurgischer Techniken basieren, sind allesamt invasiv, schmerzhaft und machen eine Schmerzbehandlung und/oder einen Narkose erforderlich.

EP 2 170 022 A1 beschreibt einen Plasma-Applikator, mit dem ein nicht thermisches Plasma auf der Haut eines Patienten appliziert werden soll. Die Anordnung wird auf die Haut eines Patienten geklebt und weist eine luftundurchlässige Hülle auf, in der sich die mit einer Hochspannungsquelle verbundene Elektrode zur Plasmaerzeugung befindet. Im Übrigen ist der Raum unter der Abdeckung mit einem luftdurchlässigen Polstermaterial ausgefüllt. Dem luftundurchlässig abgeschlossenen Raum kann über eine Gasquelle ein Gas zugeführt und über eine Gasableitung und einer Saugpumpe Gas entnommen werden. Die Haut des Patienten soll als Gegenelektrode für die Hochspannungselektrode dienen. Da das gasdurchlässige Polstermaterial einen direkten Stromfluss nicht verhindern kann, wird nicht offenbart, wie das nicht thermische Plasma gebildet und das Entstehen eines thermischen Plasmas verhindert werden soll. Ein Dielektrikum, das zu einer dielektrisch behinderten Gasentladung führen könnte, ist nicht beschrieben. Der die Umgebung der Elektrode ausfüllende gasdurchlässige Polsterstoff kann mit einer Substanz imprägniert sein, die die Ausbildung des Plasmas erleichtert und/oder eine medizinische Wirkung, insbesondere eine sterilisierende Wirkung, hat.

Weiter offenbart DE 10 2011 105 713 A1 die Merkmale des Oberbegriffs des Anspruchs 1.

Der Erfindung liegt demgegenüber die Aufgabe zugrunde, ein Gerät der eingangs erwähnten Art anzugeben, mit dem die Behandlung einer Körperoberfläche eines lebenden Körpers in verbesserter Form möglich ist.
Zur Lösung dieser Aufgabe ist ein Gerät der eingangs erwähnten Art erfindungsgemäß dadurch gekennzeichnet, dass auf der Oberfläche des Dielektrikums eine Schicht aus einer festen, offenporigen Matrix aus einem pflegenden oder heilungsfördernden Material angeordnet ist.
Das erfindungsgemäße Gerät weist auf der Oberfläche des Dielektrikums ein offenporiges Material auf, in dem sich die dielektrisch behinderte Plasmaentladung ausbilden kann. Somit ist es möglich, einerseits die Vorteile der Plasmabehandlung der Körperoberfläche auszunutzen und andererseits die Körperoberfläche im Kontakt mit dem pflegenden oder heilungsfördernden Material zu halten. Für die Durchführung der Plasmabehandlung muss daher der Kontakt mit dem pflegenden oder heilungsfördernden Material nicht aufgegeben werden, wie dies erforderlich wäre, wenn eine Plasmabehandlung und eine pflegende oder heilungsfördernde Behandlung nacheinander erfolgen würden. Insbesondere bei Wundverbänden wäre die Abnahme des Wundverbandes für die Durchführung der Plasmabehandlung u. U. sehr störend.
Das erfindungsgemäße Gerät ermöglicht somit eine kombinierte Behandlung der Körperoberfläche in Form einer, vorzugsweise wiederholt ausgeführten, Plasmabehandlung und einer Behandlung mit einem pflegenden oder heilungsfördernden Material.
Die Schicht aus dem pflegenden oder heilungsfördernden Material kann als separate Schicht mit einer zur strukturierten Oberfläche des Dielektrikums komplementären Oberfläche ausgebildet sein. Dies ist vorteilhaft, weil dadurch das Entstehen von Lufträumen vermieden wird, sodass eine einheitliche Plasmaausbildung innerhalb der Schicht erfolgt.

Die Schicht ist vorzugsweise so ausgebildet, dass sie mit dem Dielektrikum biegbar ist. Auf diese Weise bleibt der Vorteil der flexiblen Elektrodenanordnung erhalten, die mit dem Dielektrikum auch aus unregelmäßig geformte Körperpartien auflegbar ist.
Wie bei der aus DE 10 2009 060 627 A1 bekannten Elektrodenanordnung bekannt ist, kann auch für das erfindungsgemäße Gerät die Strukturierung der Oberfläche des Dielektrikums aus vorstehenden Noppen gebildet sein. Die Noppen weisen vorzugsweise eine freie ebene Stirnfläche zur Anlage an der Körperoberfläche auf.
In einer besonders bevorzugten Ausführungsform besteht die Schicht aus einem vom Körper resorbierbaren Material. Die Schicht besteht in einer bevorzugten Ausführungsform aus Collagen, sodass die Schicht vom Körper über größenordnungsmäßig einige Tage resorbiert wird. Das erfindungsgemäße Gerät bietet den Vorteil, dass auch nach der Resorption der Schicht aus dem pflegenden oder heilungsfördernden Material, insbesondere Collagen, weiterhin eine Plasmabehandlung der Körperoberfläche möglich ist. Im Fall der Behandlung einer chronischen Wunde kann somit auch nach der heilungsfördernden Resorption des Collagens ohne Veränderung der Anordnung eine Plasmabehandlung, ggf. auch wiederholt, durchgeführt werden.
Die Herstellung einer festen, flexiblen Matrix aus Collagen, wie sie für das erfindungsgemäße Gerät verwendet werden kann, ist grundsätzlich aus EP 2 322 232 A2 bekannt. Auf eine Erläuterung der Herstellung dieser Schicht kann daher hier verzichtet werden.

Weiter offenbart CA 2846 684 eine Wundauflage, welche sich insbesondere zur therapeutischen Wundversorgung eignet, wobei die Wundauflage mindestens eine luftdurchlässige Schicht mit poröser Struktur, z.B. Kollagen, umfasst.

Es ist bekannt, dass bestimmte Schichten aus einem heilenden oder pflegenden Material auch auf einen Kunststoffträger aufwachsen können, wodurch die Schicht sofort fest mit dem Kunststoffträger verbunden ist. In diesem Fall muss die Schicht daher nicht als separate Schicht hergestellt werden, sondern kann auf die strukturierte Oberfläche des Dielektrikums aufwachsen. Dies ist insbesondere für eine Collagenschicht vorteilhaft. Die aufgewachsene Schicht bildet sich dabei automatisch komplementär zu der Strukturierung der Oberfläche des Dielektrikums aus.
Die erfindungsgemäße Kombination des Geräts für die Plasmabehandlung mit einer strukturierten Dielektrikumsoberfläche mit der Anwendung einer Schicht aus heilendem oder pflegendem Material, das insbesondere vollständig vom Körper resorbierbar ist, eröffnet zahlreiche vorteilhafte Behandlungsmöglichkeiten im kosmetischen wie auch insbesondere im medizinischen Bereich. Insbesondere in Verbindung mit einer Collagenschicht lassen sich erhebliche Erfolge bei der Heilung sonst schlecht oder gar nicht verheilender Wunden erzielen.

Die Erfindung soll im Folgenden anhand eines in der Zeichnung dargestellten Ausführungsbeispiels näher erläutert werden.

Es zeigen:
- Figur 1: eine schematische Darstellung eines Geräts mit einem Dielektrikum mit strukturierter Oberfläche und einer separat erstellten Schicht, deren Oberfläche komplementär zu der Oberfläche des Dielektrikums ausgebildet ist;
- Figur 2: die Anordnung aus Figur 1 in dem zur Anwendung geeigneten zusammengefügten Zustand.

Die schematische Darstellung in der Zeichnung lässt eine flächige, flexible Elektrode 1 erkennen, die mit einer geeigneten Leitung 2 mit einer Hochspannungsquelle 3 verbunden ist. In dem dargestellten Ausführungsbeispiel liefert die Hochspannungsquelle eine Wechselspannung.

Die Elektrode ist in ein flächiges Dielektrikum 4 eingebettet und somit vollständig gegenüber der Umgebung abgeschirmt. Das flächige Dielektrikum weist somit Schmalseiten 5 und große Seitenflächen in Form einer Rückseite 6 und einer gegenüberliegenden Behandlungsseite 7 auf. Auf der Behandlungsseite 7 befinden sich aus dem quaderförmigen Körper des Dielektrikums 4 herausragende Noppen 8, die in dem dargestellten Ausführungsbeispiel zylindrisch ausgebildet sind, aber auch jede andere Form aufweisen können. Die Noppen 8 sind vorzugsweise zylindrisch ausgebildet und weisen eine gleiche Höhe auf, sodass freie, ebene Stirnseiten 9 eine gemeinsame Auflagefläche auf einer zu behandelnden Körperfläche ausbilden können. Zwischen den Noppen wird dabei ein Zwischenraum 10 in Höhe der Noppen 8 ausgebildet, wenn die Elektrodenanordnung auf einer zu behandelnden Körperoberfläche mit den Stirnseiten 9 der Noppen aufliegt.

Mit der insoweit durch DE 10 2009 060 627 A1 bekannten Elektrodenanordnung wirkt erfindungsgemäß eine Schicht 11 zusammen, die aus einer offenporigen Matrix aus einem pflegenden oder heilungsfördernden Material gebildet ist. Als pflegendes oder heilungsförderndes Material wird dabei auch eine Schicht verstanden, die als Trägerschicht mit pflegenden oder heilungsfördernden Substanzen dotiert ist.

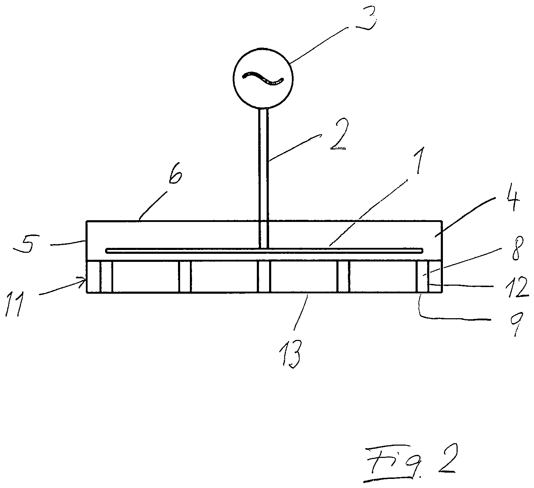

Die Schicht ist komplementär zu der zur Körperoberfläche zeigenden Oberfläche der Elektrodenanordnung ausgebildet. Demgemäß füllt die Schicht 11 den Zwischenraum 10 zwischen den Noppen 8 aus und weist zu den Noppen 8 komplementäre Öffnungen 12 auf, sodass die Schicht 11 im montierten Zustand, wie er in Figur 2 dargestellt ist, das Dielektrikum 4 zu einer flächigen Elektrodenanordnung ergänzt, die eine glatte Kontaktseite 13 zur Auflage auf der Körperoberfläche aufweist.

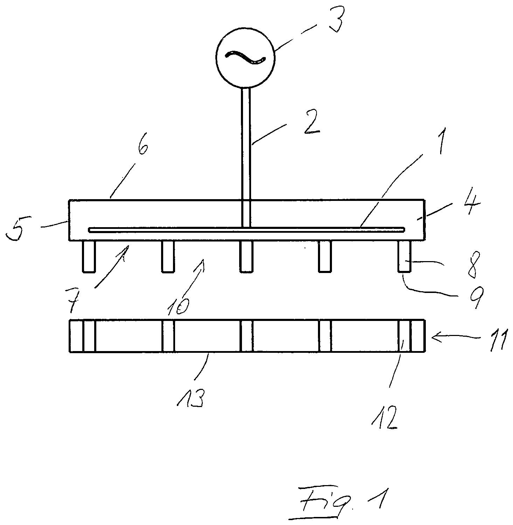

In dem dargestellten Ausführungsbeispiel entspricht die Dicke der Schicht 11 der Länge der Noppen 8. Dies ist vorteilhaft, wenn die Schicht 11 aus einem vom Körper vollständig resorbierbaren Material besteht. Im Laufe der Anwendung verschwindet somit die Schicht und die in Figur 1 separat gezeichnete Elektrodenanordnung ohne die Schicht 11 verbleibt auf der Körperoberfläche. Wenn die Dicke der Schicht 11 der Länge der Noppen 8 entspricht, fluchten die Stirnseiten 9 der Noppen 8 mit der Kontaktseite 13, liegen also bereits auf der Körperoberfläche auf, wenn die Schicht 11 noch vollständig vorhanden ist. Wird das Material der Schicht 11 vom Körper resorbiert, ändert sich somit die Lage der Elektrodenanordnung nicht.

Es ist aber auch möglich, dass die Schicht 11 dicker ausgebildet ist als die Länge der Noppen 8, sodass die Schicht 11 im Falle von zylindrischen Noppen 8 entsprechend zylindrische Sacklöcher als Öffnungen 12 aufweist. Dies ist insbesondere unproblematisch, wenn das Material der Schicht nicht resorbiert wird sondern nur pflegende oder heilungsfördernde Bestandteile enthält, die in die Körperoberfläche einwandern. Wenn die Schicht 11 aus resorbierbarem Material besteht, wird sich der Abstand des Dielektrikums 4 zur Körperober-fläche verringern, wenn die Schicht 11 durch Resorption verschwindet. In diesem Fall muss daher dafür gesorgt werden, dass eine Befestigung der Elektrodenanordnung unter einer gewissen Vorspannung auf der Körperoberfläche erfolgt, damit das Dielektrikum 4 in Richtung Körperoberfläche nachwandern kann, wenn die Schicht 11 resorbiert wird.

Das Material der Schicht 11 ist vorzugsweise eine feste Collagenmatrix, die eine Wundheilung fördert und vollständig resorbierbar ist. Die Herstellung einer derartigen Matrix ist in EP 2 322 232 A2 beschrieben, auf die verwiesen wird.

Das Dielektrikum 4 ist vorzugsweise ein gießfähiger Kunststoff, sodass die Noppen 8 einstückig an das Dielektrikum 4 angeformt werden können. Es ist allerdings auch denkbar, dass die Noppen 8 separat gefertigt und mit dem Dielektrikum 4 verbunden werden. Die Noppen 8 können dabei ein zusammenhängendes Teil des Dielektrikums 4 bilden und mit einem Gegenstück zu dem Dielektrikum 4 verbunden werden. Zweckmäßigerweise wird bei einer derartigen zweiteiligen Ausbildung die Elektrode 1 zwischen die beiden Teile des Dielektrikums 4 eingelegt werden.

Die in der Zeichnung dargestellte vollständige Einbettung der Elektrode 1 in das Dielektrikum 4 ist vorteilhaft und ermöglicht insbesondere auch die einstückige Ausbildung des Dielektrikums 4 mit den Noppen 8. Es sind aber auch Anwendungsfälle denkbar, in denen mit dem Dielektrikum 4 lediglich die Abschirmung der Elektrode 1 zur Behandlungsseite 7 hin bewirkt wird, sodass ein Überschlag von der Elektrode 1 zur Körperoberfläche mit Sicherheit unterbunden wird. Die Abdeckung der Elektrode 1 zur Rückseite 6 hin kann das ggf. unterbleiben oder eine Isolierung auf andere Weise erfolgen.

Die dargestellten Noppen 8 sind nur eine denkbare Ausführungsform der Strukturierung der Behandlungsseite 7 des Dielektrikums 4. Es sind beliebige Strukturierungen, beispielsweise in Form von parallelen Rillen, gitterförmigen Einkerbungen o. ä. möglich, um einen für die Plasmaentladung benötigten Zwischenraum 10 auszubilden. Die Schicht 11, die komplementär zur Oberfläche des Dielektrikums 4 ausgebildet ist und den Zwischenraum 10 vollständig ausfüllt, bildet aufgrund ihrer Offenporigkeit Kanäle zwischen dem Dielektrikum 4 und der Körperoberfläche aus, in denen die dielektrisch behinderte Plasmaentladung zwischen der Elektrode 1 und der Körperoberfläche als Gegenelektrode stattfindet. Die Plasmaentladung wird somit innerhalb der in dem Material der Schicht 11 vorhandenen Kanäle durchgeführt.

Die Strukturierung der Behandlungsseite 7 des Dielektrikums muss dabei auch nicht in einem regelmäßigen Muster erfolgen. Wichtig ist lediglich eine über die aktive Fläche auf der Behandlungsseite 7 statistisch gleichmäßige Verteilung des Zwischenraums 10, damit es über die Behandlungsfläche zu einer gleichmäßigen Plasmaausbildung kommt.

## Patentansprüche

1. Gerät zur Behandlung einer Körperoberfläche eines lebenden Körpers mittels eines dielektrisch behinderten Plasmas, mit einem flächigen, flexiblen Dielektrikum (4), das eine an eine Hochspannungsquelle (3) angeschlossene Elektrode (1) von der Körperoberfläche abschirmt und zur Anlage an der als Gegenelektrode fungierenden Körperoberfläche geeignet ist, und mit einer strukturierten Oberfläche (7) ausgebildet ist, die einen Gasraum für die Plasmaentladung zwischen Dielektrikum (4) und Körperoberfläche ermöglicht, **dadurch gekennzeichnet, dass** auf der strukturierten Oberfläche (7) des Dielektrikums (4) eine Schicht (11) aus einer festen, offenporigen Matrix aus einem pflegenden oder heilungsfördernden Material angeordnet ist.

2. Gerät nach Anspruch 1, **dadurch gekennzeichnet, dass** die Schicht (11) als separate Schicht mit einer zur strukturierten Oberfläche des Dielektrikums (4) komplementären Oberfläche ausgebildet ist.

3. Gerät nach Anspruch 1 oder 2, **dadurch gekennzeichnet, dass** die Schicht (11) mit dem Dielektrikum (4) biegbar ist.

4. Gerät nach einem der Ansprüche 1 bis 3, **dadurch gekennzeichnet, dass** die Strukturierung der Oberfläche des Dielektrikums (4) aus vorstehenden Noppen (8) gebildet ist.

5. Gerät nach Anspruch 4, **dadurch gekennzeichnet, dass** die Noppen (8) eine freie ebene Stirnseite (9) zur Anlage an der Körperoberfläche aufweisen.

6. Gerät nach einem der Ansprüche 1 bis 5, **dadurch gekennzeichnet, dass** die Schicht (11) aus einem vom Körper resorbierbarem Material besteht.

7. Gerät nach Anspruch 6, **dadurch gekennzeichnet, dass** die Schicht (11) aus Collagen besteht.

8. Gerät nach einem der Ansprüche 1 und 3 bis 7, **dadurch gekennzeichnet, dass** die Schicht (11) als Beschichtung des Dielektrikums (4) gebildet ist.

9. Gerät nach Anspruch 8, **dadurch gekennzeichnet, dass** die Schicht durch Aufwachsen auf dem Dielektrikum (4) gebildet ist.

10. Gerät nach Anspruch 4 oder 5, **dadurch gekennzeichnet, dass** die Dicke der Schicht (11) der Länge der vorstehenden Noppen (8) entspricht.

## Claims

1. A device for treating a body surface of a living body by means of a dielectric barrier discharge plasma, comprising a planar, flexible dielectric (4), which shields an electrode (1) connected to a high-voltage source (3) from the body surface and which is suitable for placement on the body surface acting as a counter electrode and which is embodied with a structured surface (7) enabling a gas space for the plasma discharge between the dielectric (4) and the body surface, **characterized in that** a layer (11) made of a solid, open-pore matrix of a curative or healing material is arranged on the structured surface (7) of the dielectric (4).

2. The device as claimed in claim 1, **characterized in that** the layer (11) is embodied as a separate layer with a surface that is complementary to the structured surface of the dielectric (4).

3. The device as claimed in claim 1 or 2, **characterized in that** the layer (11) is bendable with the dielectric (4).

4. The device as claimed in one of claims 1 to 3, **characterized in that** the structuring of the surface of the dielectric (4) is formed from protruding bumps (8).

5. The device as claimed in claim 4, **characterized in that** the bumps (8) have a free planar end side (9) for placement on the body surface.

6. The device as claimed in one of claims 1 to 5, **characterized in that** the layer (11) consists of material that is resorbable by the body.

7. The device as claimed in claim 6, **characterized in that** the layer (11) consists of collagen.

8. The device as claimed in one of claims 1 and 3 to 7, **characterized in that** the layer (11) is formed as a coating of the dielectric (4).

9. The device as claimed in claim 8, **characterized in that** the layer is formed by growth on the dielectric (4).

10. The device as claimed in claim 4 or 5, **characterized in that** the thickness of the layer (11) corresponds to the length of the protruding bumps (8).

## Revendications

1. Appareil pour le traitement d'une surface corporelle d'un corps vivant au moyen d'un plasma entravé de manière diélectrique, comprenant un diélectrique surfacique flexible (4), qui constitue pour une électrode (1) branchée à une source de haute tension (3) un écran vis-à-vis de la surface corporelle et qui convient pour venir en contact avec la surface corporelle qui fait office d'électrode antagoniste, et comportant une surface (7) réalisée structurée, qui permet de ménager un volume de gaz pour la décharge du plasma entre le diélectrique (4) et la surface corporelle,
**caractérisé en ce qu'**une couche (11) d'une matrice solide à pores ouverts en un matériau favorisant les soins ou la guérison est agencée sur la surface structurée (7) du diélectrique (4).

2. Appareil selon la revendication 1, **caractérisé en ce que** la couche (11) est réalisée sous forme de couche séparée avec une surface complémentaire de la surface structurée du diélectrique (4).

3. Appareil selon la revendication 1 ou 2, **caractérisé en ce que** la couche (11) est capable d'être cintrée avec le diélectrique (4).

4. Appareil selon l'une des revendications 1 à 3, **caractérisé en ce que** la structure de la surface du diélectrique (4) est constituée par des boutons en saillie (8).

5. Appareil selon la revendication 4, **caractérisé en ce que** les boutons (8) présentent une face frontale libre plane (9) pour la venue en contact contre la surface corporelle.

6. Appareil selon l'une des revendications 1 à 5, **caractérisé en ce que** la couche (11) est en un matériau résorbable par le corps.

7. Appareil selon la revendication 6, **caractérisé en ce que** la couche (11) est en collagène.

8. Appareil selon l'une des revendications 1 et 3 à 7, **caractérisé en ce que** la couche (11) est formée à titre de revêtement du diélectrique (4).

9. Appareil selon la revendication 8, **caractérisé en ce que** la couche est formée par croissance sur le diélectrique (4).

10. Appareil selon la revendication 4 ou 5, **caractérisé en ce que** l'épaisseur de la couche (11) correspond à la longueur des boutons en saillie (8).
